# EUROPÄISCHE PATENTSCHRIFT

(11) **EP 1 962 916 B1**
(45) Veröffentlichungstag und Bekanntmachung des Hinweises auf die Patenterteilung: **06.05.2009**
(21) Anmeldenummer: 06829604.5
(22) Anmeldetag: 14.12.2006
(51) Int. Cl.: A61L 24/02, A61L 27/42

(54) **BIOKOMPATIBLER MAGNESIUMWERKSTOFF**
BIOCOMPATIBLE MAGNESIUM MATERIAL
MATERIAU BIOCOMPATIBLE A BASE DE MAGNESIUM

(30) Priorität: 14.12.2005 DE 102005060203
(43) Veröffentlichungstag der Anmeldung: 03.09.2008
(73) Patentinhaber: GKSS-Forschungszentrum Geesthacht GmbH, 21502 Geesthacht (DE)
(72) Erfinder: KAINER, Ulrich, 21522 Hohnstorf (DE); DIERINGA, Hajo, 21394 Südergellersen (DE); HORT, Norbert, 21339 Lüneburg (DE)
(74) Vertreter: UEXKÜLL & STOLBERG
(86) Internationale Anmeldenummer: PCT/EP2006/012050
(87) Internationale Veröffentlichungsnummer: WO 2007/068479

(56) Entgegenhaltungen:
- WO-A-02/16286
- WO-A-20/04101017

## Beschreibung

Die vorliegende Erfindung betrifft ein Verfahren zur Herstellung eines biokompatiblen Werkstoffs, aus dem Strukturen zur Fixierung von Knochenbrüchen oder -schäden gefertigt werden können.

Knochen stellen einen gradierenden Werkstoff dar. Das Bedeutet, dass sich die Eigenschaften, insbesondere die Porösitäten, lokalstetig ändern. Ein abrupter Sprung in den Eigenschaften, der zu mechanischer Instabilität an der Grenzfläche (Corticalis Spongiosa) führen würde, wird vermieden. Ein optimales Knochenersatzmaterial sollte daher diese gradierte Struktur nachahmen, um die gewünschten Eigenschaften wie mechanische Stabilität, Degradationsgrad, Porösität unter lokaler Variation zur Verfügung zu stellen. Andererseits sind bioresorbierbare oder biodegradierbare Implantate im Bereich der Knochenrekonstruktion wünschenswert, die sich nach Abheilen des Schadens von alleine auflösen und damit auf einen zweiten Eingriff zur Explantation verzichtet werden kann. Ein derartiges biodegradierbares Implantat aus einem biodegradierbarem Metall ist aus der DE 197 31 021 bekannt.

Ein solcher Implantat-Werkstoff muss dabei eine ausreichende mechanische Stabilität aufweisen und die Biodegradation muss in einer mit dem Knochenheilungsprozess synchronisierten Abbaugeschwindigkeit erfolgen. Bioresorbierbare Polymerimplantate werden beispielsweise als Alternative zu Titan eingesetzt. Die zurzeit wichtigste Gruppe der resorbierbaren synthetischorganischen Materialien sind die linearen, aliphatischen Polyester, insbesondere die auf Milch- und Glykolsäure basierenden Polylaktide und -glykolide. Diese Materialien behalten ihre Festigkeit während des Heilungsprozesses bei und Zerfallen durch Hydrolyse langsam zu Milchsäure. Auf Grund ihrer eingeschränkten mechanischen Stabilität werden sie jedoch bevorzugt zur Versorgung nicht-lasttragender Knochensegmente verwendet.

Im Bereich der synthetischen, anorganischen Knochenersatzmaterialien gibt es Bemühungen, Gerüste insbesondere aus keramischen Knochenersatzmaterialien zur Knochenregeneration anzubieten, in die das Knochengewebe hineinwachsen kann. Diese können jedoch auf Grund der Sprödigkeit der mechanischen Werkstoffe größere mechanische Belastungen nicht aufnehmen. Um die mechanische Festigkeit und Belastbarkeit dieser Gerüste aus keramischen Materialien zu erhöhen, werden sogenannte Kompositmaterialien verwendet.

Auch biodegradierbare metallische Implantatwerkstoffe wie Magnesiumlegierungen, bieten eine gewisse mechanische Stabilität und werden daher zunehmend interessanter. Derartige Implantatwerkstoffe sind in der US-A-3 687 135 und der DE-A-102 53 634 beschrieben. Diese Materialien sind jedoch nicht biokompatibel, d.h. vollständig biologisch verträglich.

Aufgabe der vorliegenden Erfindung ist es, ein Verfahren zur Herstellung eines biokompatiblen Werkstoffs bereit zu stellen, aus dem massive Strukturen wie beispielsweise Schrauben oder Platten gefertigt werden, die zur Fixierung von Knochenbrüchen oder -schäden Verwendung finden und eine ausreichende mechanische Stabilität aufweisen. Die vorliegende Aufgabe wird durch ein Verfahren gelöst, bei dem zunächst ein Gemisch aus Apatit und einer Magnesiumlegierung in Form von Spänen oder Pulver in einer Kugelmühle gemahlen wird, bis ein homogenes Gemisch entsteht. Die homogene Mischung wird in einem zweiten Schritt konsolidiert. Dies kann durch Strangpressen oder Schmieden erfolgen. Aus dem erhaltenen massiven Material kann dann durch spanende Bearbeitung die gewünschte Form herausgearbeitet werden.

Die Aufgabe wird ebenfalls durch einen biokompatiblen, zur Fixierung von Knochenbrüchen und -schäden geeigneten Werkstoff gelöst, der ein homogenes Gemisch aus Apatit und einer Magnesiumlegierung enthält.

Die Magnesiumlegierung enthält vorzugsweise Aluminium, besonders bevorzugt in einer Menge von 0 bis 15 Gew.%, bevorzugter 1 bis 10 Gew.%. Sie kann ferner Zink, vorzugsweise in einer Menge von 0 bis 7 Gew.%, besonders bevorzugt 1 bis 5 Gew.%, Zinn, vorzugsweise in einer Menge von 0 bis 6 Gew.%, besonders bevorzugt 1 bis 4 Gew.%, Lithium, vorzugsweise in einer Menge von 0 bis 5 Gew.%, besonders bevorzugt 0,5 bis 4 Gew.%, Mangan, vorzugsweise in einer Menge von 0 bis 5 Gew.%, besonders bevorzugt 1 bis 4 Gew.%, Silizium, vorzugsweise in einer Menge von 0 bis 5 Gew.%, besonders bevorzugt 1 bis 4 Gew.%, Calcium, vorzugsweise in einer Menge von 0 bis 3 Gew.%, besonders bevorzugt in einer Menge von 1 bis 3 Gew.%, Yttrium, vorzugsweise in einer Menge von 0 bis 5 Gew.%, besonders bevorzugt in einer Menge von 0,5 bis 4 Gew.%, Strontium, vorzugsweise in einer Menge von 0 bis 4 Gew.%, besonders bevorzugt 0,1 bis 3 Gew.%, ein oder mehrere Metalle, ausgewählt aus der Gruppe der seltenen Erden, vorzugsweise in einer Menge von 0 bis 5 Gew.%, besonders bevorzugt in einer Menge von 0,1 bis 3 Gew.%, Silber, vorzugsweise in einer Menge von 0 bis 2 Gew.%, besonders bevorzugt 0,1 bis 2 Gew.%, Eisen, vorzugsweise in einer Menge von 0 bis 0,1 Gew.%, Nickel, vorzugsweise in einer Menge von 0 bis 0,1 Gew.% und/oder Kupfer, vorzugsweise in einer Menge von 0 bis 0,1 Gew.% enthalten.

Das bevorzugte Gewichtsverhältnis von Apatit zu Magnesiumlegierung beträgt 100:1 bis 1:100, bevorzugter 20:1 bis 1:20 und insbesondere 1:5 bis 5:1.

Es wurde gefunden, dass man ein im Vergleich mit der Matrixlegierung verfestigtes Gefüge aus Legierung und Apatit-Teilchen erhält, bei dem in der metallischen Matrix die nicht-metallischen Apatit-Teilchen feindispers verteilt sind. Implantate aus diesem Werkstoff bieten gegenüber den bekannten biodegradierbaren Implantaten vor allem eine höhere mechanische Stabilität. Die Magnesiumlegierung wird nach und nach korrosiv abgebaut. Die feinverteilten Apatitanteile werden auf diese Weise über einen längeren Zeitraum freigesetzt und unterstützen das Körpergewebe bei der Heilung bzw. beim Knochenwachstum. Da neben den beschriebenen Eigenschaften auch die Festigkeit eine wichtige Rolle spielt, ist bei diesem Werkstoff durch die feinverteilten nicht-metallischen Bestandteile in der metallischen Matrix-eine Dispersionsverfestigung gegeben. Das bedeutet im Vergleich mit der Matrixlegierung ist der Werkstoff signifikant verfestigt. Schrauben und Platten, die aus diesem Werkstoff gefestigt werden, weisen gegenüber unverstärkten Magnesiumlegierungen, die als Korrosiv abbaubare Werkstoffe auch ohne Apatitanteil als Implantate verwendet werden könnten, eine Festigkeitssteigerung auf.

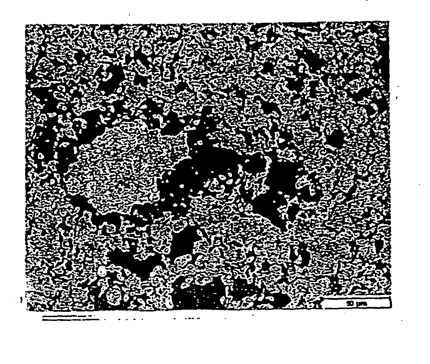

Figur 1 ist eine lichtmikroskopische Aufnahme der Mikrostruktur des Materials. Dunkel ist das eingelagerte Apatit. Hell ist die Magensiummatrix. Man kann erkennen, dass das Apatit homogen in der Magnesiummatrix dispergiert ist.

## Patentansprüche

1. Werkstoff zur Fixierung von Knochenbrüchen und/oder -schäden, der ein homogenes Gemisch aus Apatit und einer Magnesiumlegierung enthält.

2. Werkstoff nach Anspruch 1, **dadurch gekennzeichnet, dass** die Magnesiumlegierung Aluminium enthält.

3. Werkstoff nach Anspruch 2, **dadurch gekennzeichnet, dass** der Gehalt an Aluminium in der Magnesiumlegierung 0 bis 15 Gew.-% beträgt.

4. Werkstoff nach einem der Ansprüche 1 bis 3, **dadurch gekennzeichnet, dass** die Magnesiumlegierung Zink enthält.

5. Werkstoff nach Anspruch 4, **dadurch gekennzeichnet, dass** der Gehalt an Zink in der Magnesiumlegierung 0 bis 7 Gew.-% beträgt.

6. Werkstoff nach einem der Ansprüche 1 bis 3, **dadurch gekennzeichnet, dass** die Magnesiumlegierung Zinn enthält.

7. Werkstoff nach Anspruch 4, **dadurch gekennzeichnet, dass** der Gehalt an Zinn in der Magnesiumlegierung 0 bis 6 Gew.-% beträgt.

8. Werkstoff nach einem der Ansprüche 1 bis 7, **dadurch gekennzeichnet, dass** die Magnesiumlegierung Zink enthält.

9. Werkstoff nach Anspruch 8, **dadurch gekennzeichnet, dass** der Gehalt an Zink in der Magnesiumlegierung 0 bis 7 Gew.-% beträgt.

10. Werkstoff nach einem der Ansprüche 1 bis 9, **dadurch gekennzeichnet, dass** die Magnesiumlegierung Lithium enthält.

11. Werkstoff nach Anspruch 10, **dadurch gekennzeichnet, dass** der Gehalt an Lithium in der Magnesiumlegierung 0 bis 5 Gew.-% beträgt.

12. Werkstoff nach einem der Ansprüche 1 bis 11, **dadurch gekennzeichnet, dass** die Magnesiumlegierung Mangan enthält.

13. Werkstoff nach Anspruch 12, **dadurch gekennzeichnet, dass** der Gehalt an Mangan in der Magnesiumlegierung 0 bis 5 Gew.-% beträgt.

14. Werkstoff nach einem der Ansprüche 1 bis 13, **dadurch gekennzeichnet, dass** die Magnesiumlegierung Yttrium enthält.

15. Werkstoff nach Anspruch 14, **dadurch gekennzeichnet, dass** der Gehalt an Yttrium in der Magnesiumlegierung 0 bis 5 Gew.-% beträgt.

16. Werkstoff nach einem der Ansprüche 1 bis 15, **dadurch gekennzeichnet, dass** die Magnesiumlegierung ein Metall aus der Gruppe der seltenen Erden enthält.

17. Werkstoff nach Anspruch 16, **dadurch gekennzeichnet, dass** der Gehalt an seltenen Erden in der Magnesiumlegierung 0 bis 5 Gew.-% beträgt.

18. Werkstoff nach einem der Ansprüche 1 bis 17, **dadurch gekennzeichnet, dass** das Gewichtsverhältnis von Apatit zu Magnesiumlegierung 1:100 bis 100:1 beträgt.

19. Werkstoff nach Anspruch 18, **dadurch gekennzeichnet, dass** das Gewichtsverhältnis von Apatit zu Magnesiumlegierung 1:20 bis 20:1 beträgt.

20. Werkstoff nach Anspruch 18, **dadurch gekennzeichnet, dass** das Gewichtsverhältnis von Apatit zu Magnesiumlegierung 1:5 bis 5:1 beträgt.

21. Verfahren zur Herstellung eines biokompatiblen Werkstoffs zur Fixierung von Knochenbrüchen und/oder -schäden gemäß einem der Ansprüche 1 bis 20, bei dem:
ein Gemisch aus Apatit und einer Magnesiumlegierung in Form von Spänen in einer Kugelmühle gemahlen wird, bis ein homogenes Gemisch entsteht, und das homogene Gemisch in einem zweiten Schritt konsolidiert wird.

22. Biokompatibler Werkstoff gemäß einem der Ansprüche 1 bis 20 zur Verwendung zur Fixierung von Knochenbrüchen und/oder -schäden.

## Claims

1. Material for fixing bone fractures and/or damage which contains a homogeneous mixture of apatite and a magnesium alloy.

2. Material according to claim 1, **characterized in that** the magnesium alloy contains aluminium.

3. Material according to claim 2, **characterized in that** the aluminium content of the magnesium alloy is 0 to 15 wt.-%.

4. Material according to one of claims 1 to 3, **characterized in that** the magnesium alloy contains zinc.

5. Material according to claim 4, **characterized in that** the zinc content of the magnesium alloy is 0 to 7 wt.-%.

6. Material according to one of claims 1 to 3, **characterized in that** the magnesium alloy contains tin.

7. Material according to claim 4, **characterized in that** the tin content of the magnesium alloy is 0 to 6 wt.-%.

8. Material according to one of claims 1 to 7, **characterized in that** the magnesium alloy contains zinc.

9. Material according to claim 8, **characterized in that** the zinc content of the magnesium alloy is 0 to 7 wt.-%.

10. Material according to one of claims 1 to 9, **characterized in that** the magnesium alloy contains lithium.

11. Material according to claim 10, **characterized in that** the lithium content of the magnesium alloy is 0 to 5 wt.-%.

12. Material according to one of claims 1 to 11, **characterized in that** the magnesium alloy contains manganese.

13. Material according to claim 12, **characterized in that** the manganese content of the magnesium alloy is 0 to 5 wt.-%.

14. Material according to one of claims 1 to 13, **characterized in that** the magnesium alloy contains yttrium.

15. Material according to claim 14, **characterized in that** the yttrium content of the magnesium alloy is 0 to 5 wt.-%.

16. Material according to one of claims 1 to 15, **characterized in that** the magnesium alloy contains a metal from the group of the rare earths.

17. Material according to claim 16, **characterized in that** the rare earths content of the magnesium alloy is 0 to 5 wt.-%.

18. Material according to one of claims 1 to 17, **characterized in that** the weight ratio of apatite to magnesium alloy is 1:100 to 100:1.

19. Material according to claim 18, **characterized in that** the weight ratio of apatite to magnesium alloy is 1:20 to 20:1.

20. Material according to claim 18, **characterized in that** the weight ratio of apatite to magnesium alloy is 1:5 to 5:1.

21. Process for the production of a biocompatible material for fixing bone fractures and/or damage according to one of claims 1 to 20, in which:
a mixture of apatite and a magnesium alloy in the form of chips is ground in a ball mill until a homogeneous mixture forms, and the homogeneous mixture is consolidated in a second step.

22. A biocompatible material according to one of claims 1 to 20 for use for fixing bone fractures and/or damage.

## Revendications

1. Matériau de fixation de fractures osseuses et/ou de lésions osseuses qui contient un mélange homogène d'apatite et d'un alliage de magnésium.

2. Matériau selon la revendication 1, **caractérisé en ce que** l'alliage de magnésium contient de l'aluminium.

3. Matériau selon la revendication 2, **caractérisé en ce que** la teneur en aluminium dans l'alliage de magnésium est de 0 à 15 % en poids.

4. Matériau selon l'une quelconque des revendications 1 à 3, **caractérisé en ce que** l'alliage de magnésium contient du zinc.

5. Matériau selon la revendication 4, **caractérisé en ce que** la teneur en zinc dans l'alliage de magnésium est de 0 à 7 % en poids.

6. Matériau selon l'une quelconque des revendications 1 à 3, **caractérisé en ce que** l'alliage de magnésium contient de l'étain.

7. Matériau selon la revendication 4, **caractérisé en ce que** la teneur en étain dans l'alliage de magnésium est de 0 à 6 % en poids.

8. Matériau selon l'une quelconque des revendications 1 à 7, **caractérisé en ce que** l'alliage de magnésium contient du zinc.

9. Matériau selon la revendication 8, **caractérisé en ce que** la teneur en zinc dans l'alliage de magnésium est de 0 à 7 % en poids.

10. Matériau selon l'une quelconque des revendications 1 à 9, **caractérisé en ce que** l'alliage de magnésium contient du lithium.

11. Matériau selon la revendication 10, **caractérisé en ce que** la teneur en lithium dans l'alliage de magnésium est de 0 à 5 % en poids.

12. Matériau selon l'une quelconque des revendications 1 à 11, **caractérisé en ce que** l'alliage de magnésium contient du manganèse.

13. Matériau selon la revendication 12, **caractérisé en ce** la teneur en manganèse dans l'alliage de magnésium est de 0 à 5 % en poids.

14. Matériau selon l'une quelconque des revendications 1 à 13, **caractérisé en ce que** l'alliage de magnésium contient de l'yttrium.

15. Matériau selon la revendication 14, **caractérisé en ce** la teneur en yttrium dans l'alliage de magnésium est de 0 à 5 % en poids.

16. Matériau selon l'une quelconque des revendications 1 à 15, **caractérisé en ce que** l'alliage de magnésium contient un métal du groupe des terres rares.

17. Matériau selon la revendication 16, **caractérisé en ce que** la teneur en terres rares dans l'alliage de magnésium est de 0 à 5 % en poids.

18. Matériau selon l'une quelconque des revendications 1 à 17, **caractérisé en ce que** le rapport en poids de l'apatite à l'alliage de magnésium est de 1:100 à 100:1.

19. Matériau selon la revendication 18, **caractérisé en ce que** le rapport en poids de l'apatite à l'alliage de magnésium est de 1:20 à 20:1.

20. Matériau selon la revendication 18, **caractérisé en ce que** le rapport en poids de l'apatite à l'alliage de magnésium est de 1:5 à 5:1.

21. Procédé de production d'un matériau biocompatible pour la fixation de fractures osseuses et/ou de lésions osseuses selon l'une quelconque des revendications 1 à 20 dans lequel :
un mélange d'apatite et d'un alliage de magnésium est broyé sous forme de copeaux dans un broyeur à billes jusqu'à ce qu'un mélange homogène soit obtenu et le mélange homogène est consolidé dans une deuxième étape.

22. Matériau biocompatible selon l'une quelconque des revendications 1 à 20 à utiliser pour fixer des fractures osseuses et/ou des lésions osseuses.
